# EUROPÄISCHE PATENTSCHRIFT

(11) **EP 0 264 008 B1**
(45) Veröffentlichungstag und Bekanntmachung des Hinweises auf die Patenterteilung: **26.01.1994**
(21) Anmeldenummer: 87114023.2
(22) Anmeldetag: 25.09.1987
(51) Int. Cl.: C07D 257/04

(54) **Verfahren zur Herstellung von 5-Methyltetrazol**
Process for the preparation of 5-methyl tetrazole
Procédé de préparation de 5-méthyltétranole

(30) Priorität: 11.10.1986 DE 3634717
(43) Veröffentlichungstag der Anmeldung: 20.04.1988
(73) Patentinhaber: Dynamit Nobel Aktiengesellschaft, 53839 Troisdorf (DE)
(72) Erfinder: Bison, Günter, D-5210 Troisdorf 5 (DE); Schlupp, Johannes, Dr., D-5060 Bergisch-Gladbach (DE); Winterscheid, Josef, D-5205 St. Augustin 1 (DE); Thewalt, Klaus, Dr., D-5810 Witten (DE)

(56) Entgegenhaltungen:
- FR-A- 2 517 676
- US-A- 4 097 479

## Beschreibung

Die Erfindung betrifft ein Verfahren zur industriellen Herstellung von 5-Methyltetrazol der Formel:

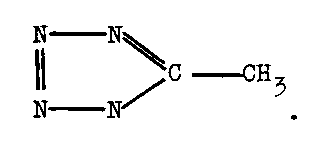

5-Methyltetrazol dient als pharmazeutisches Zwischenprodukt, insbesondere zum Aufbau von Antibiotica.

Nach dem Stand der Technik ist die Herstellung von 5-Methyltetrazol aus Acetonitril, Thioazidamid, Acetamidrazonhydrochlorid oder 1-Ethoxy-1,1-diazidoethan bekannt. Diese Herstellverfahren haben den Nachteil unbefriedigender Ausbeuten, teils schwer zugängige Ausgangsstoffe und der Gefährlichkeit der Reaktion bei Verwendung von Stickstoffwasserstoffsäure. Durch ungenügende Ausbeuten kommt es zur Bildung zersetzlicher und gefährlicher Nebenprodukte, die nur schwer und aufwendig zu entfernen sind.

Die Herstellung von 5-Methyltetrazol durch Einwirkung von Stickstoffwasserstoffsäure auf Acetonitril verläuft langsam und ergibt nur Ausbeuten von etwa 50 %. Nach DE-PS 962 798 kann 5-Methyltetrazol aus Thioacetamid - anstelle des reaktionsträgen Acetonitrils - in Tetrahydrofuran als Lösemittel durch Umsetzung mit Aluminiumazid in einer Gesamtausbeute von 63 % erhalten werden. Diese Verwendung von Aluminiumazid anstelle von Natriumazid ist aber aufwendig, da nur eines von drei gebundenen Azidionen zur Reaktion ausgenutzt wird.

Finnegan et al (J. AM. Chem. Soc. 80, 3908 (1958) berichtet, daß man bei Synthese von 5-substituierten Tetrazole durch Umsetzung eines organischen Nitrils mit einem Azid in Dimethylformamid oder Dimethylsulfoxid in Gegenwart eines Katalysators höhere Ausbeuten erzielt als bei Verwendung anderer Lösungsmittel. Zu den Katalysatoren zählt beispielsweise Ammoniumchlorid. Dieses führt jedoch zur Bildung von Ammoniumazid, das an kühlen Stellen,z.B. am Kühler, ein leicht zersetzliches Sublimat bildet. Freie Amine verwendet Finnegan nicht.

Nach DE-OS 28 09 798 wird versucht, diese Nachteile der Herstellung von 5-substituierten Tetrazolen durch Umsetzung eines Nitrils mit einem Alkaliazid oder Ammoniumazid in Gegenwart von Monoalkylaminen oder Dialkylaminen in Gegenwart der zugehörigen Säureaddition Salze des Amins zu lösen. Ausbeuten von 60 bis 70 % sind jedoch nur für das System Morpholin/Morpholin · HCl angegeben.

Wendet man dieses Verfahren zur Herstellung 5-Methyltetrazol an, so erhält man das Produkt (vgl. Vergleichsbeispiel ) nur in geringer Ausbeute. Die Bildung von Begleitprodukten erfordert aufwendige Reinigungsoperationen, so daß das Verfahren umständlich und unwirtschaftlich ist.

Es fehlt somit ein wirtschaftlich industrielles Verfahren, um gefahrlos und in hoher Ausbeute 5-Methyltetrazol herzustellen. Der Erfindung liegt daher diese Aufgabe zugrunde.

Es wurde nun gefunden, daß bei Verwendung von besonders Triethylamin als Verdünnungsmittel, Suspensionsmittel oder Lösungsmittel und in Gegenwart von Triethylamin-Hydrochlorid das 5-Methyltetrazol überraschend bei Umsatz von Acetonitril mit Alkaliazid oder ggf.Ammoniumazid in der sehr hohen Ausbeute von über 95 % und in Reinheiten von über 98 % anfällt. Ausbeuten von weit über 99 % und ebenso hohe Reinheiten sind erreichbar. Allgemein können Trialkylamine und die zugehörigen Hydrochloride der jeweils verwendeten Amine mit Kettenlängen von C₁ bis C₄ verwendet werden, von denen Triethylamin sehr bevorzugt und Tripropylamin bevorzugt ist. Sehr bevorzugt ist die Abwesenheit weiterer Lösungsmittel. Umständliche Reinigungsoperationen sind nicht mehr erforderlich.

Das Verfahren kann infolge eines nur geringen sich aufbauenden Überdrucks von etwa 3 bar bei Reaktionstemperaturen von etwa 105 bis 115°C in den üblich vorhandenen Rührwerksapparaturen durchgeführt werden, so daß man keine speziellen Druckgefäße benötigt. Geeignete Amine sind dann besonders Triethylamin und ggf. Tripropylamin.

Temperaturen von 90 bis 160° C sind zweckmäßig, bevorzugt sind 110 bis 135° C. Die Reaktionspartner werden im molaren Verhältnis oder bevorzugt mit einem Überschuß des Azids von 0,02 bis 15,und sehr bevorzugt 2,0 bis 12 Mol-% eingesetzt. Das Hydrochlorid kann in molarer Menge oder im Überschuß von 0,02 bis 10 Mol-%, bezogen auf das Azid, eingesetzt werden.

Das beim Entfernen des Trialkylamins in Gegenwart von Alkalihydroxid zunächst entstehende Alkalisalz des 5-Methyltetrazols ist fast gänzlich frei von Nebenprodukten. Die Freisetzung des 5-Methyltetrazols mit Salzsäure erfolgt daher ohne die bisherige Gefährdung durch Nebenprodukte und deren Zersetzung. Das Produkt kann kristallin oder in Lösung organischer Lösungsmittel der Verwendung zugeführt werden.

### Vergleichsversuch nach DE-OS 28 09 798

Acetonitril 20,5 g (0,5 Mole),
Morpholinhydrochlorid 67,9 g (0,55 Mole),
Natriumazid 35,8 g (0,55 Mole)
wurden in 332 ml Morpholin suspendiert und 13 Stunden auf 130° C erhitzt.

Nach Abkühlen der Reaktionsmischung auf 15° C wurde 28,7 g = 89,3 % des ausgeschiedenen Natriumchlorid entfernt. Die dunkel gefärbte Reaktionslösung wurde im Wasserstrahlvakuum eingeengt, der Rückstand in Wasser aufgenommen, der pH mit 10 Gew.-%iger Natronlauge auf pH 13 angehoben. Anschließend wird die Lösung mit Filterhilfsmitteln geklärt und eingeengt. Dieser Reinigungsvorgang des Aufnehmens des Rückstands in Wasser und Klären mit Aktivkohle wurde zweimal wiederholt.

Der nun verbleibende Rückstand wurde in einer Soxhletapparatur mit Aceton extrahiert, anschließend das Aceton im Rotationsverdampfer entfernt und das erhaltene Produkt aus n-Butylacetat umkristallisiert.

### Beispiel 1

In einem Rührwerksautoklaven von 1 l Inhalt wurden
20,5 g (0,5 Mol) Acetonitril,
75,7 g (0,55 Mol) Triethylaminhydrochlorid,
35,8 g (0,55 Mol) Natriumazid in
418 ml Triethylamin suspendiert und 6 Stunden auf 130° C erhitzt.

Nach dem Abkühlen wurde bei 50° C im Wasserstrahlvakuum das Triethylamin weitgehend abdestilliert. Der Destillationsrückstand wurde in Wasser gelöst, mit NaOH (10 Gew.-%) auf pH 12,8 gebracht und Triethylamin und Wasser im Wasserstrahlvakuum entfernt. Der gefärbte Rückstand wurde in 250 ml Wasser gelöst, mit konzentrierter Salzsäure auf pH 1,2 gebracht und die Lösung mit Aktivkohle geklärt. Die Lösung wurde mit Isobutanol aceotrop entwässert und ausgeschiedenes Natriumchlorid entfernt. Nach Abdestillieren des Isobutanols wurden 41,9 g kristallines Produkt vom Fp 139-42° C (Reinheit 98 %) erhalten. Ausbeute, bezogen auf eingesetztes Acetonitril, 98,1 % d.Th.).

### Beispiel 2

In einem emaillierten Rührwerksbehälter von 800 l Inhalt wurden
20,5 kg (0,50 Kmol) Acetonitril,
75,0 kg (0,59 Kmol) Triethylaminhydrochlorid,
35,8 kg (0,55 Kmol) Natriumazid in
304 kg Triethylamin vorgelegt und das Gemisch 6 Stunden auf 115° C erhitzt, wobei im Reaktor ein Druck von 4 bar entstand. Nach Abkühlen auf 20° C und Entspannen des Reaktors entstanden zwei Schichten. Die Triethylamin-Schicht wurde abgetrennt. Die das Produkt enthaltende Schicht mit 100 l Wasser versetzt und die Suspension mit 10 Gew.-%iger Natronlauge auf pH 12,9 eingestellt. Das freigesetzte Triethylamin wurde im Wasserstrahlvakuum abdestilliert, der Rückstand mit 100 l Wasser versetzt und mit konz. HCl auf pH 1,3 gebracht. Die Lösung wurde mit Aktivkohle geklärt, worauf Wasser abdestilliert und restliche Anteile aceotrop mit Isobutanol entfernt wurden. Aus dieser Lösung wurde ausgeschiedenes Natriumchlorid mit einer Drucknutsche entfernt und das Produkt wie in Beispiel 1 isoliert.

Es wurden 42,4 kg Produkt (Fp 139-42° C) mit einer Reinheit von 98,7 % erhalten. Ausbeute 99,8 % d. Th.

### Beispiel 3

Beispiel 1 wird wiederholt, wobei jedoch 0,55 Mol Tripropylamin . HCl und 400 ml Tripropylamin verwendet werden. Ausbeute und Reinheit entsprechen Beispiel 1.

### Beispiel 4

Beispiel 1 wird wiederholt, wobei jedoch 0,53 Mol Ammoniumazid verwendet wurde und die Reaktionstemperatur 120° C betrug. Ausbeute und Reinheit entsprechen Beispiel 1.

## Patentansprüche

1. Verfahren zur Herstellung von 5-Methyltetrazol durch Umsetzung von Acetonitril mit einem Ammonium- oder Alkaliazid in Gegenwart eines Amins und dessen HCl-Additionssalzes, **dadurch gekennzeichnet**, daß ein Trialkylamin mit 1 bis 4 Kohlenstoffatomen als Lösungsmittel und ein Trialkylaminhydrochlorid mit 1 bis 4 Kohlenstoffatomen als Katalysator, bei 90 bis 160°C und einem sich dabei in einer üblichen Rührwerksapparatur aufbauenden geringen Überdruck, eingesetzt wird.

2. Verfahren nach Anspruch 1, dadurch gekennzeichnet, daß die Reaktionstemperatur 110 bis 135°C, beträgt.

3. Verfahren nach einem der Ansprüche 1 oder 2, dadurch gekennzeichnet, daß die Reaktionspartner im molaren Verhältnis eingesetzt werden.

4. Verfahren nach einem der Ansprüche 1 oder 2, dadurch gekennzeichnet, daß der Überschuß an Ammonium- oder Alkaliazid 0,02 bis 15, vorzugsweise 2 bis 12 Mol-% beträgt.

5. Verfahren nach einem der Ansprüche 1 oder 2, dadurch gekennzeichnet, daß der Überschuß an Trialkylaminhydrochlorid 0,02 bis 10 Mol-%, bezogen auf das Azid, beträgt.

## Claims

1. Process for the production of 5-methyltetrazole by reaction of acetonitrile with an ammonium or alkali azide in the presence of an amine and its HCl-addition salt, characterised in that there is employed at 90 to 160°C and a slight pressure which is increasing in a conventional apparatus equipped with stirrer, a trialkylamine with 1 to 4 carbon atoms as solvent and a trialkylamine hydrochloride with 1 to 4 carbon atoms as catalyst.

2. Process according to claim 1, characterised in that the reaction temperature amounts to 110 to 135°C.

3. Process according to one of claims 1 or 2, characterised in that the reaction partners are employed in the molar ratio.

4. Process according to one of claims 1 or 2, characterised in that the excess of ammonium or alkali azide amounts to 0.02 to 15, preferably 2 to 12 mol%.

5. Process according to one of claims 1 or 2, characterised in that the excess of trialkylamine hydrochloride amounts to 0.02 to 10 mol%, related to the azide.

## Revendications

1. Procédé de préparation du 5-méthyltétrazole par réaction de l'acétonitrile avec un azoture d'ammonium ou de métal alcalin, en présence d'une amine et de son chlorhydrate, **caractérisé** en ce que l'on utilise une trialkylamine ayant 1 à 4 atomes de carbone dans les chaînes alkyle en tant que solvant, et un chlorhydrate de trialkylamine ayant 1 à 4 atomes de carbone dans les chaînes alkyle en tant que catalyseur, en opérant à une température de 90 à 160°C et avec une faible surpression qui s'établit alors dans un appareil classique à agitateur.

2. Procédé selon la revendication 1, **caractérisé** en ce que la température de réaction atteint 110 à 135°C.

3. Procédé selon la revendication 1 ou 2, **caractérisé** en ce que les partenaires de la réaction sont utilisés suivant le rapport molaire.

4. Procédé selon la revendication 1 ou 2, **caractérisé** en ce que l'excès d'azoture d'ammonium ou de métal alcalin atteint 0,02 à 15 % en moles, de préférence 2 à 12 % en moles.

5. Procédé selon la revendication 1 ou 2, **caractérisé** en ce que l'excès de chlorhydrate de trialkylamine atteint 0,02 à 10 % en moles, par rapport à l'azoture.
